(19) **Europäisches Patentamt**
**European Patent Office**
**Office européen des brevets**

(11) **EP 3 906 001 B1**

(12) **EUROPEAN PATENT SPECIFICATION**

(45) Date of publication and mention
of the grant of the patent:
**19.11.2025  Bulletin 2025/47**

(21) Application number: **19907314.9**

(22) Date of filing: **19.11.2019**

(51) International Patent Classification (IPC):
*A61F 13/15* (2006.01)    *A61F 13/49* (2006.01)

(52) Cooperative Patent Classification (CPC):
**A61F 13/84;** A61F 2013/8497

(86) International application number:
**PCT/CN2019/119401**

(87) International publication number:
**WO 2020/140637 (09.07.2020 Gazette 2020/28)**

(54) **ABSORBENT ARTICLE HAVING ARTWORK**

SAUGFÄHIGER ARTIKEL MIT ILLUSTRATIONEN

ARTICLE ABSORBANT COMPORTANT UNE ILLUSTRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **05.01.2019   PCT/CN2019/070520**

(43) Date of publication of application:
**10.11.2021   Bulletin 2021/45**

(73) Proprietor: **The Procter & Gamble Company
Cincinnati, OH 45202 (US)**

(72) Inventors:
• **WANG, Xuechun
Beijing 101312 (CN)**
• **LIU, Li
Beijing 101312 (CN)**
• **MORIMOTO, Koichi
Beijing 101312 (CN)**

• **ZHANG, Jing
Beijing 101312 (CN)**
• **WOLFE, Sarah Nicole
Cincinnati, Ohio 45202 (US)**
• **TOURNOUX, Monica Renee
Columbus, Ohio 43231 (US)**

(74) Representative: **P&G Patent Germany
Procter & Gamble Service GmbH
Sulzbacher Straße 40
65824 Schwalbach am Taunus (DE)**

(56) References cited:
**EP-A1- 1 139 955       EP-A1- 2 596 715
WO-A1-00/35401         WO-A1-2014/200119
CN-A- 101 193 617      CN-A- 102 014 823
CN-A- 102 573 742      CN-A- 103 313 686
CN-A- 104 349 761      US-A1- 2007 250 406**

**Description**

FIELD OF THE INVENTION

The present invention relates to absorbent articles having artwork which provides an integral appearance with the wearer when worn.

BACKGROUND OF THE INVENTION

Absorbent articles such as diapers may be grouped into two major groups based on difference in application method, tape type and pant type. Tape type articles are applied by engaging fastening means which typically comprise the combination of a hook member and a receiving member. Pant type articles are applied by inserting the wearer's legs into the leg openings and sliding up the article. In either application method, diaper change may be an unpleasant time for the wearer and/or caregiver. On the other hand, diaper change may provide the opportunity for communication between the wearer and caregiver.

Absorbent articles may be provided with printed artwork to make the article attractive to the wearer and/or the caregiver. Particularly for young children, attractive artwork on the article may provide a positive developmental effect, such as enhancing communication opportunity with the caregiver during diaper change. Attractive artwork may be those that connote an undergarment look, are in clear color and shape, or showing characters and objects in noticeable size, or provide an interactive element with certain structure of the absorbent article. Further, attractive artwork may connote high quality of the article not only upon purchase or upon taking out from the package, but also when the article is worn by the wearer.

WO0035401A1 relates to a refastenable pant with graphics hidden during use and in a prefastened condition, which become visible only when the fasteners are disengaged from one another. The hidden graphics can be used by the caregiver or parent as a motivational and educational instrument to improve the speed and quality of the total toilet training process.

EP2596715A1 discloses a nonwoven web with zigzag unit patterns, which can be used as a receiving component/member of a fastener. The fastening system may be provided with visual indicia or graphics to aid in the alignment of the fasteners of the fastening system

EP1139955A1 relates to pant-like disposable absorbent article including a fastening system with at least one fastening component disposed in one waist region and a pair of side attachment panels disposed in the other waist region that are adapted to releasably engage the at least one fastening component. In form of a training pant, the article may include graphics to be aesthetically and/or functionally pleasing to the wearer and the caregiver. WO2018/152831A1 discloses wearable articles having an elastic belt region having force profiles.

Based on the foregoing, there is a need for an absorbent article with attractive artwork which may provide a unique appearance when worn, or opportunity of communication between the wearer and caregiver. There is also a need for providing such an absorbent article without compromise to the performance as an absorbent article, such as fit, wearability, and containment performance. There is further a need for providing such an absorbent article in an economical manner.

SUMMARY OF THE INVENTION

The invention provides an absorbent article as defined in the claims.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

Figure 1A is a schematic perspective view of one embodiment of a tape article of the present invention in a worn, yet unfastened, state showing the front to side region.
Figure 1B is a schematic perspective view of Figure 1B in a fastened state.
Figure 2A is a schematic plan view of one embodiment of a tape type article of the present invention in an unfastened state showing the garment-facing surface.
Figure 2B is a partial schematic plan view of Figure 2A in a fastened state showing the garment-facing surface.
Figure 3A is a schematic plan view of one embodiment of a tape type article of the present invention in an unfastened state showing the garment-facing surface.

Figure 3B is a partial schematic plan view of Figure 3A in a fastened state showing the garment-facing surface.

Figure 4A is a schematic plan view of one embodiment of a tape type article of the present invention in an unfastened state showing the garment-facing surface.

Figure 4B is a partial schematic plan view of Figure 4A in a fastened state showing the garment-facing surface.

Figure 5 is a schematic perspective view of one embodiment of a pant type article of the present invention.

Figure 6A is a partial schematic plan view of one embodiment of a pant type article of the present invention showing the front side garment-facing surface.

Figure 6B is a partial schematic plan view of one embodiment of a pant type article of the present invention showing the back side garment-facing surface.

Figure 7A is a partial schematic plan view of one embodiment of a pant type article of the present invention showing the front side garment-facing surface.

Figure 7B is a partial schematic plan view of one embodiment of a pant type article of the present invention showing the back side garment-facing surface.

Figure 7C is a schematic perspective view of Figure 7A in a worn state showing the front to side region.

Figure 7D is a schematic perspective view of Figure 7B in a worn state showing the back to side region.

Figure 7E is a schematic perspective view according to the prior art as worn on a wearer showing the back to side region.

Figure 8 is a schematic side plan view of one embodiment of a wearable article of the present invention in a flat uncontracted condition showing the garment facing surface.

Figure 9 is a side view of one embodiment of a pant type article of the present invention in a worn state.

Figure 10 is a schematic view of an example of a hanger-type sample holding fixture according to the "Whole Article Force Measurement".

Figure 11 is a side view of one embodiment of a pant type article of the present invention worn on a stretch board according to the "Belt Seam Shape Measurement".

Figures 12A1 - 12F2 are synthetic photographs of pant type articles utilized as visual presentations in the Examples section.

DEFINITIONS

As used herein, the following terms shall have the meaning specified thereafter:
"Absorbent article" refers to articles of wear which may be in the form of taped diapers, pant diapers, incontinent briefs, feminine hygiene garments, and the like. The "absorbent article" may be so configured to absorb and contain various exudates such as urine, feces, and menses discharged from the body.

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having an L* value of at least 94, an a* value equal to 0 $\pm$ 2, and a b* value equal to 0 $\pm$ 2 according to the "Color Measurement" described below.

DETAILED DESCRIPTION OF THE INVENTION

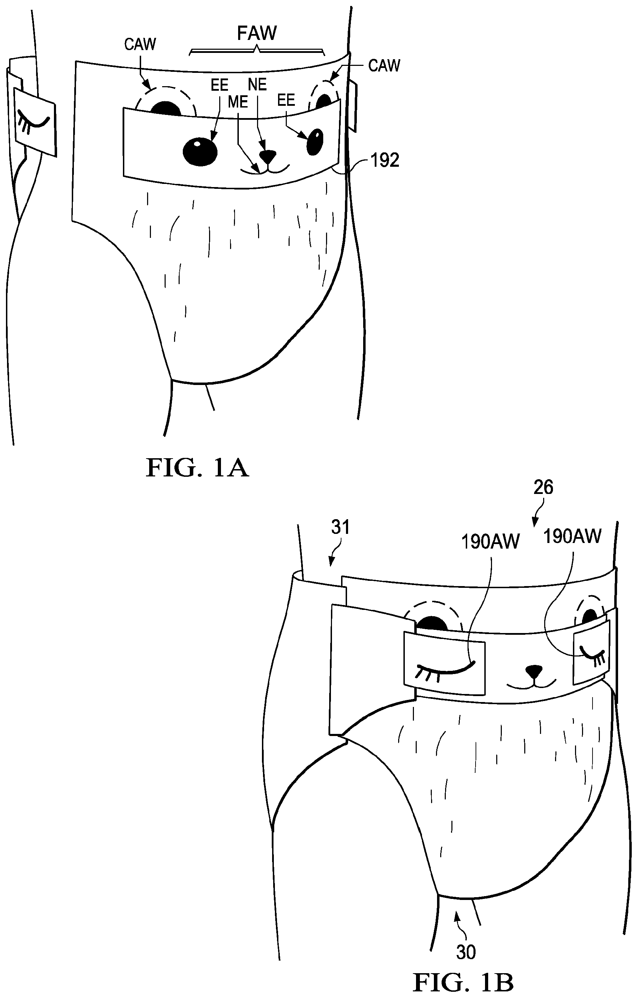

Referring to Figures 1A, 1B, 3A and 5, the present invention is related to an absorbent article having a longitudinal direction along the longitudinal axis L1 and a transverse direction along the transverse axis T1, the article having a front region 26, a back region 28, a side region 31, and a crotch region 30; the article comprising an absorbent chassis 38 and an application means; the absorbent chassis 38 comprising a liquid permeable topsheet, a liquid impermeable backsheet, and an absorbent material existing region 62 disposed between the topsheet and the backsheet; the application means enabling the absorbent article to be applied by providing a waist opening and a pair of leg openings, the longitudinal dimension of the article matching the leg openings defined as the crotch region 30, wherein the remainder of the article is the front region 26 and the back region 28. The absorbent material existing region 62 is the region wherein a certain stiffness and opacity is provided to the article, and thus artwork visibility is greater than the other regions. At least some of the front region 26 and at least some of the back region 28 may form a side region 31 when worn on the wearer. The region visible from the side of the wearer when the article is worn is defined as the side region 31.

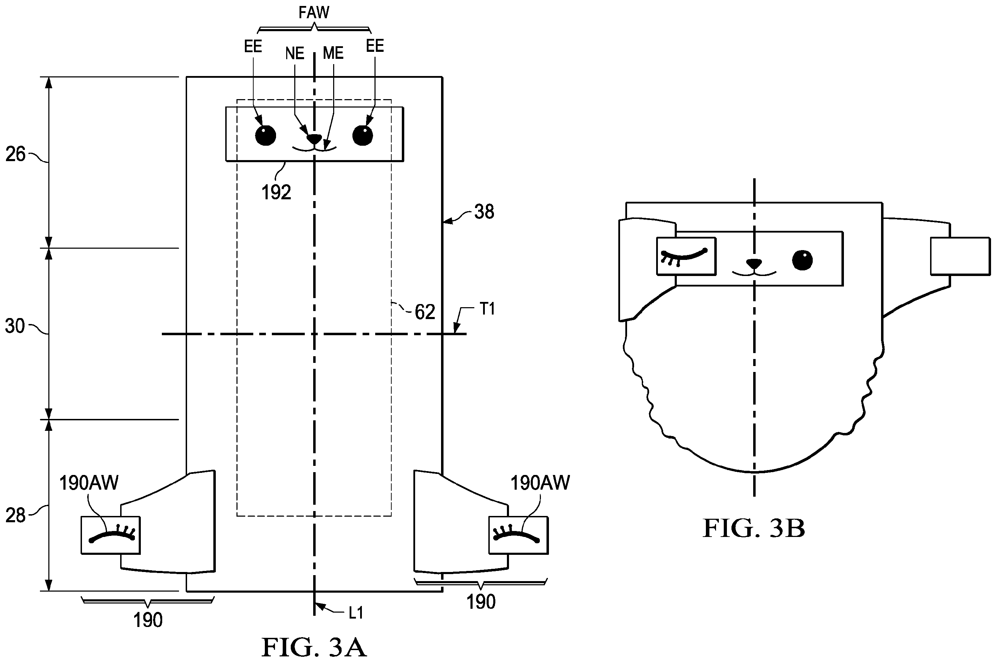

The article of the present invention may be that of the taped type as in Figure 3A wherein the application means is a fastening system comprising a pair of elongate members 190 and a receiving member 192, the elongate members 190 transversely protruding from the back region 28 of the absorbent chassis 38 and fastenable with the receiving member 192 disposed on the front region 26, wherein the eye elements EE are superposed with the receiving member 192. For the tape type article of the present invention, the front, crotch, and back regions 26, 28, 30 are roughly one thirds of the longitudinal dimension of the article. Referring to Figure 1B in the fastened state, the side region 31 comprises at least some portion of the elongate members 190.

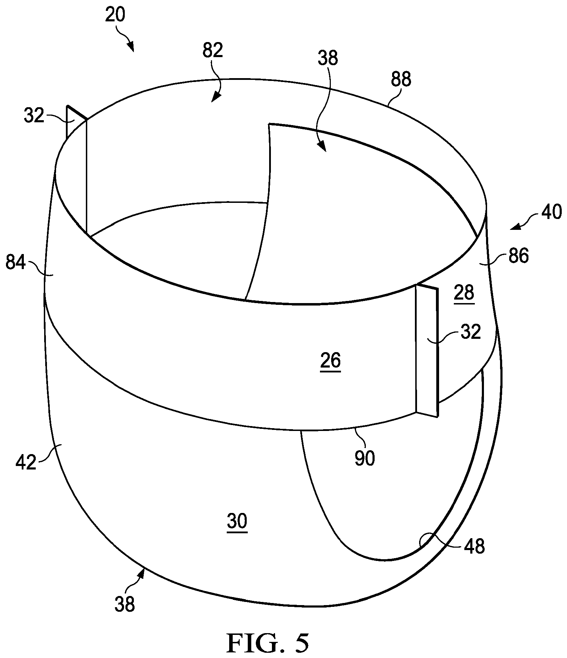

The article of the present invention may be that of the pant type as in Figure 5 wherein the application means is an elastic belt 40 extending transversely from the front and back regions 26, 28 of the absorbent chassis 38 and seamed with each other at the pair of transverse edges as side seams 32. For the pant type article of the present invention, the front and back regions 26, 28 are the regions extending in the transverse direction where the side seams 32 exists. The side region 31 comprises the side seams 32.

Referring to Figures 1A, 2A, 3A, 4A, 6A, 7A and 7C, whether the taped type or pant type, the article of the present invention comprises a facial artwork FAW visible from the garment facing side disposed at least partially in the front region 26, the facial artwork FAW comprising a pair of eye elements EE disposed in the front region 26, wherein the eye elements EE are superposed with the absorbent material existing region 62. What is defined as the facial artwork FAW herein are the eye elements EE, nose element NE, and mouth element ME of an animal. The facial artwork FAW herein is not intended to include other elements of the face or head. The article with artwork of the present invention provide an integral appearance of a three-dimensional article when worn on the wearer, in particular, a young wearer up to about 96 months old, or about 48 months old. The artwork are so configured such that when worn on the wearer, a good portion of the facial artwork FAW, and particularly the eye elements EE, are matched with the position in which the wearer of the front side torso shows greater projection. Thus, the article as worn provides an integral appearance in combination with the three-dimensional body shape of the wearer. The facial artwork FAW may resemble a first animal. What is meant by animal herein may be a character resembling a real animal, or a fictious animal, or a fictious human figure.

The eye elements EE may be provided in a color that is clearly distinguishable from the base color of the article, while maintaining a soft appearance connoting high quality. The base color of the article may be the color of the material, for example nonwoven substrate, of the article. The base color of the article may be a specific color provided for the front region 26 of the article. When the garment facing side of the front region 26 has a base color, the base color having an LAB value of $L_1$, $A_1$, and $B_1$, the eye elements EE have an eye color, the eye color having an LAB value of $L_2$, $A_2$, and $B_2$, the $\Delta E$ between the base color and the eye color is at least about 10, preferably from about 10 to about 50 based on the following formula, according to measurements herein.

$$\sqrt{(L_1 - L_2)^2 + (A_1 - A_2)^2 + (B_1 - B_2)^2}$$

The article of the present invention, when worn on a wearer, provides an integral appearance of a three dimension article with the wearer by the front region 26 having the facial artwork FAW comprising eye elements EE, the side region 31, and the back region 28. Such integral appearance of the artwork with the wearer may provide pleasure of child bearing or care giving to the caregiver, as it transforms an otherwise two-dimensional article into a three-dimensional character in combination with the three-dimensional body shape of the wearer. The present artwork may enhance communication between the wearer and caregiver during diaper change, or promote diaper change, by providing the opportunity to give the wearer an experience as though the wearer were to be part of the first animal. Enhancing communication may include, for example, story telling of the wearer transforming into the first animal.

Referring to Figures 1A, 2A and 7A, the article of the present invention may further comprise a contour artwork CAW visible from the garment facing side disposed at least partially in the front region 26. What is defined the contour artwork CAW is artwork belonging to the first animal visible from the front side of the article when worn, except for the facial artwork FAW. The contour artwork CAW may include a head, a hair, a mane, a horn, a spike, a cockscomb, a feather, an eyebrow, an ear, a cheek, a set of whiskers, a neck, or other elements. The contour may be expressed in lines or by a boarder created by difference of color. The contour artwork CAW is disposed outside of the periphery of the facial artwork FAW, and may extend surrounding the facial artwork FAW to a certain extent, or may substantially surround the facial artwork FAW, however, the contour artwork CAW does not include shoulders, the torso, or other elements of the first animal extending beyond the neck of the animal. By limiting the facial artwork FAW and the contour artwork CAW to body parts of the first animal belonging to the neck and above, the integral appearance of the artwork worn on the wearer is enhanced.

The contour artwork CAW may substantially surround the facial artwork FAW, namely the contour artwork CAW may comprise a devoid area DA of less than about 90 degrees, or less than about 60 degrees from the middlepoint EEC of the eye elements EE, or from about 30 degrees to less than 90 degrees, according to measurements herein. By having very little devoid area DA, the facial artwork FAW may be emphasized.

The devoid area DA may be disposed between the facial artwork FAW and the longitudinal center of the article. The devoid area DA may be disposed toward the transverse periphery of the eye elements EE. The combination of the facial artwork FAW and the contour artwork CAW may express a facial appearance looking to the side (not shown), rather than front. Providing a devoid area DA toward the transverse periphery of the eye elements EE may be beneficial for describing a facial expression looking towards the side.

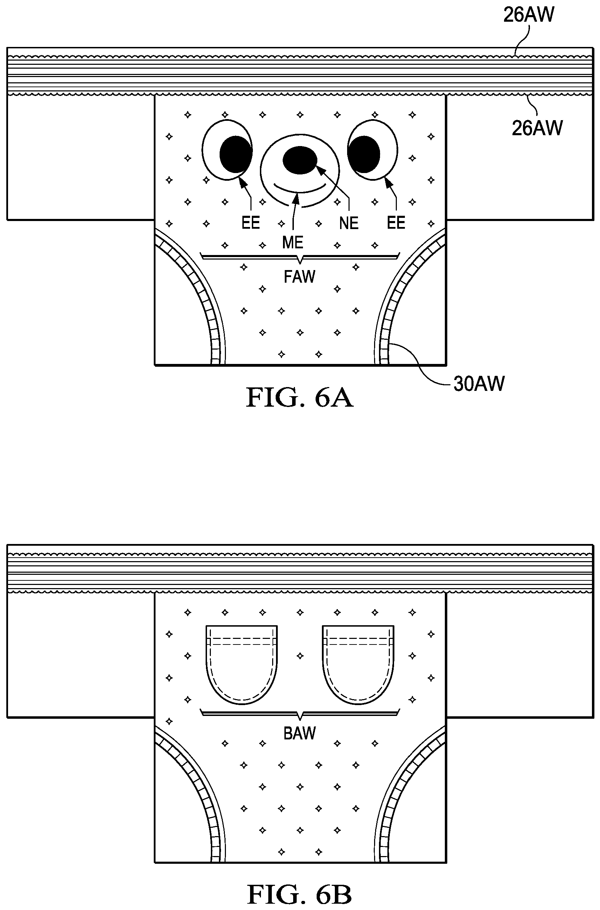

The contour artwork CAW may comprise a devoid area DA of at least about 90 degrees, or from about 90 degrees to about 270 degrees from the middlepoint EEC of the eye elements EE, according to measurements herein. By having such devoid area DA, the integral appearance is not discontinued by the contour. There may be no contour artwork CAW, namely the first animal visible from the front side may be solely expressed by the facial artwork FAW, namely having a devoid area DA of 360 degrees, as in Figure 6A.

The facial artwork FAW is visible from the garment facing side and visible from the front as worn on the wearer. Thus, the facial artwork FAW may be disposed at least partially in the front region 26, and the eye elements EE are disposed in the front region 26. While the other facial artwork FAW and contour artwork CAW may extend into the crotch region 30, they are also preferably visible from the front as worn on the wearer.

The eye elements EE have a dimension of from about 30% to about 120%, or from about 50% to about 110%, or from about 65% to about 120%, or from about 70% to about 100% of the transverse dimension of the absorbent material existing region 62. By providing the eye elements EE in such transverse dimension, the integral appearance of the artwork with the wearer is enhanced.

The front region 26 may be disposed with other front artwork 26AW other than those described above. The crotch region 30 may be disposed with other crotch artwork 30AW other than those described above. The other front artwork 26AW and the other crotch artwork 30AW are those that do not belong to the first animal. Other front artwork 26AW may include accessory on the head or hair: such as a bow, a ribbon, a hat, a cap, or other elements. The other front artwork 26AW and other crotch artwork 30AW may include geometric patterns and shapes that may be coordinated with the color or shape of elements of the first animal.

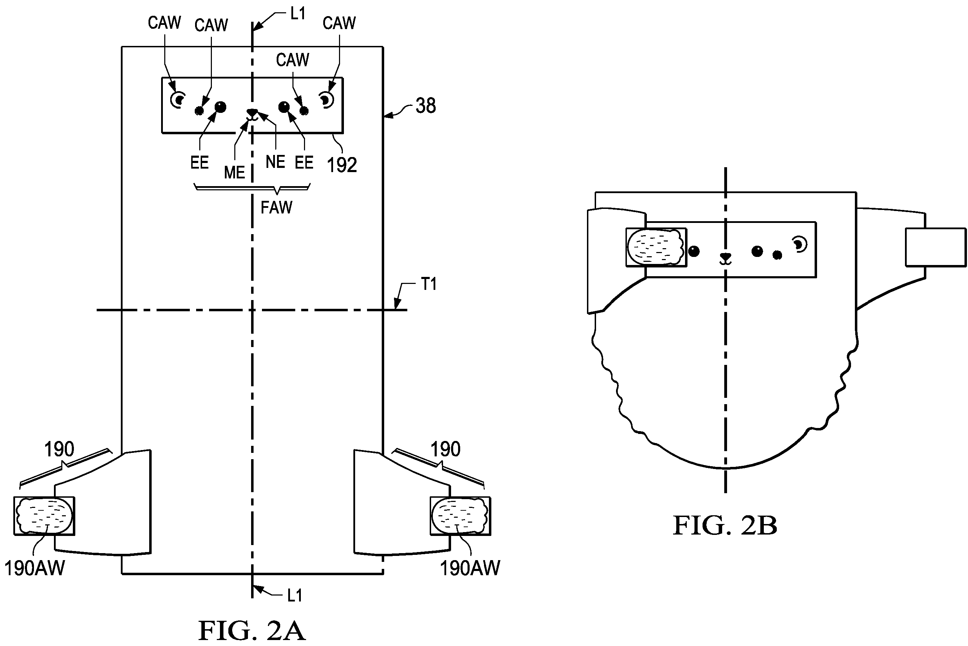

Referring to Figures 1A, 1B, 2A, 3A, and 4A, the article of the present invention may comprise a fastening system comprising a pair of elongate members 190 and a receiving member 192, the elongate members 190 transversely protruding from the back region 28 of the absorbent chassis 38 and fastenable with the receiving member 192 disposed on the front region 26, wherein the eye elements EE are superposed with the receiving member 192. The elongate members 190 may comprise a portion equipped with hook members. The receiving member 192 may have loop members which engage with the hook members, or made integral with the remainder of the garment facing surface of the article, wherein such garment facing surface is selected from a substrate which engages with the hook members. Referring to Figure 2A, the contour artwork CAW may be disposed in paired elements on the receiving member 192, the paired elements of contour artwork CAW disposed symmetrically in view of the longitudinal axis L1.

The eye elements EE combined with the contour artwork CAW, disposed symmetrically in view of the longitudinal axis, may serve as a fitting guide on the receiving member 192. The fitting guide enables the caregiver to position the elongate members 190 to be engaged symmetrically on the receiving member 192 to ensure secure fit around both the left and right legs. The fitting guide may also serve as a fit indicator which indicates if the article is of a suitable size for the wearer, and which may indicate the size change or growth of the wearer over time. For example as in Figure 2A, the narrow fitting guide may be the eye elements EE, the intermediate fitting guide may be the contour artwork CAW resembling cheeks, and the wide fitting guide may be the contour artwork CAW resembling ears of the first animal.

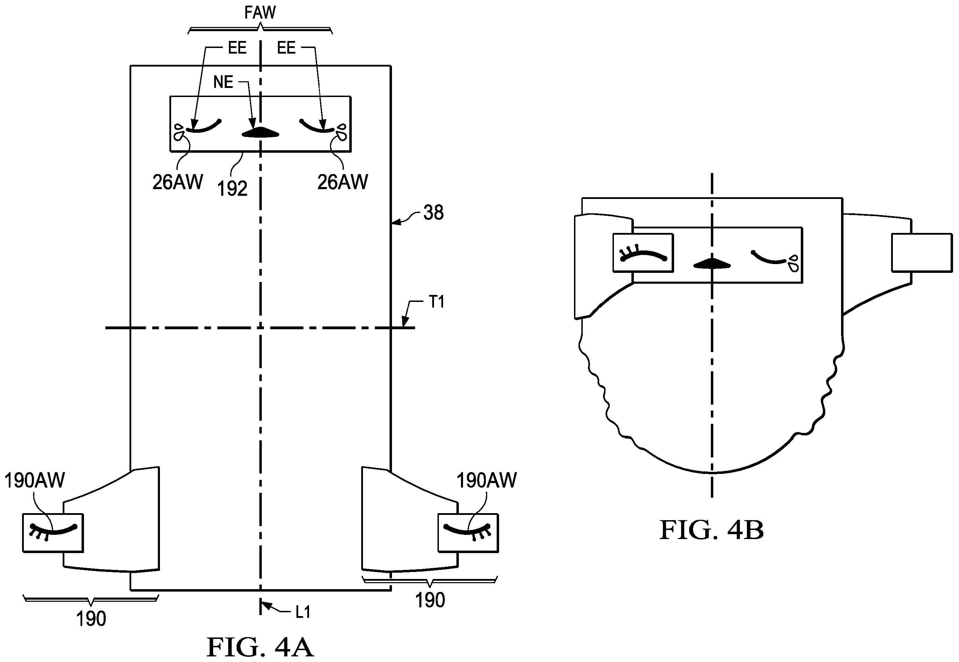

Referring to Figures 1A, 1B, 2A, 2B, 3A, 3B, 4A, and 4B, the elongate members 190 may comprise a covering artwork 190AW, the covering artwork 190AW configured to be able to superpose one or both of the eye elements EE and contour artwork CAW and visible from the garment facing side when fastened. The covering artwork 190AW may be a body element, such as hands, paws or wings, or a graspable object such as a hat, or a cap, configured to appear as covering one or both of the eye elements EE and contour artwork CAW. For example as in Figures 2A and 2B, the covering artwork 190AW may be a pair of paws covering the eye elements EE or the cheek or ear contour artwork CAW. The covering artwork 190AW may be a different eye expression compared to the eye elements EE. For example as in Figures 3A and 3B, the covering artwork 190AW may be a pair of closed eyes covering the eye elements EE in awakened expression, resembling the act of going to sleep before the last diaper change. For example as in Figures 4A and 4B, the covering artwork 190AW may be a pair of smiling eyes which may cover the eye elements EE in crying expression, resembling the act of feeling better after crying. Such interactive artwork may also enhance positive development effect.

The present invention may also be a merchandisable package comprising a plurality of absorbent articles as describe above. The merchandisable package may comprise a first article and a second article, wherein the second article has at least one of the eye elements EE and the covering artwork 190AW different from the first article. For example, a first article may be provided with eye elements EE in an awakened expression and the second article may be provided with the eye element in a sleeping expression. Such choice of article may provide pleasure of the caregiver when changing the diaper in the morning and before going to bed. For example, a first article and a second article may be provided with the same eye elements EE, but having different covering artwork 190AW. For example, the first and second article may be provided with the same covering artwork 190AW, but with different facial artwork FAW describing different facial expression selected from, for example, crying and smiling. Such choice of article may provide intuitive communication between the wearer and caregiver, and may support regulating life rhythm.

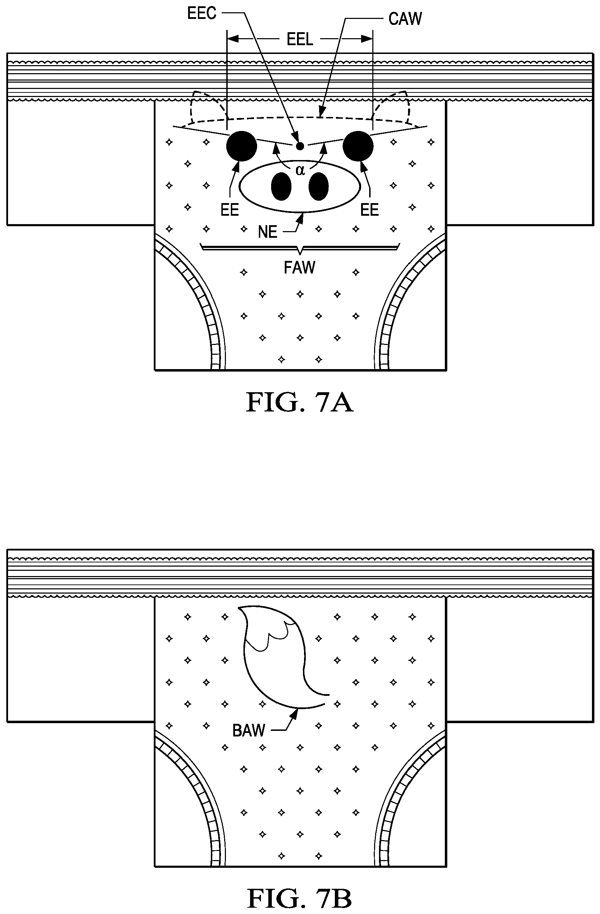

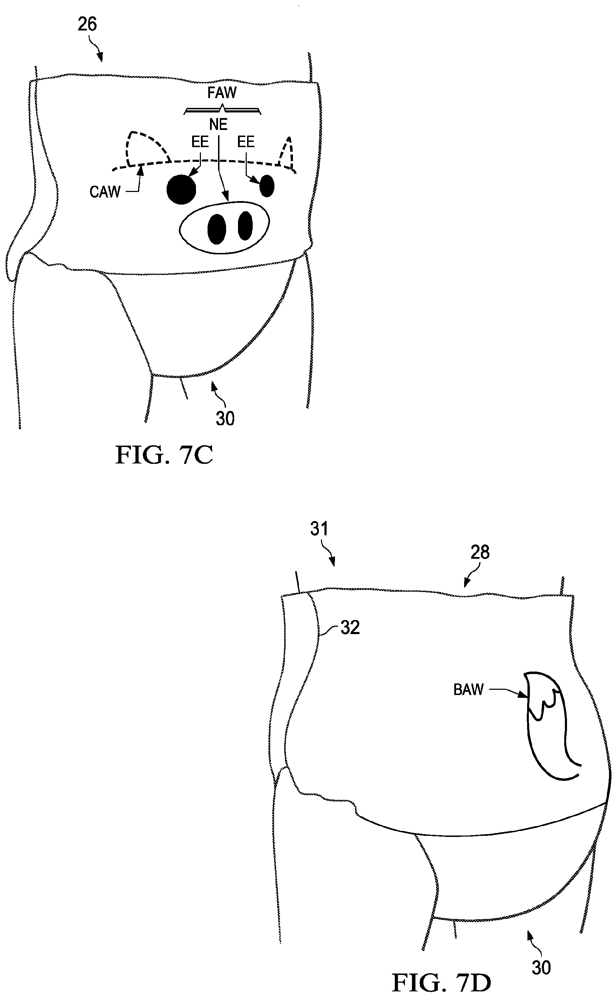

Referring to Figures 6B, 7B, and 7D, the article of the present invention has the back region 28 comprising a buttock artwork BAW visible from the garment facing side disposed in the back region 28, wherein the buttock artwork BAW is superposed with the absorbent material existing region 62. What is defined as a buttock artwork BAW herein is an animal part or a pair of pockets visible from the garment facing side as worn by the wearer and disposed at least partially in the back region 28. The buttock artwork BAW may include a part of the tail, a part of the bottom connected with the tail, a pair of wings, a pair of pockets or other elements. The artwork are also configured such that when worn on the wearer, a good portion of the buttock artwork BAW are matched with the position in which the wearer of the back side torso shows greater projection. Thus, in combination with the facial artwork FAW, the article as worn provides an integral appearance with the wearer. The buttock artwork BAW may resemble a first animal, or a second animal of different species, or a pair of pockets. By providing the facial artwork FAW and buttock artwork BAW coming from elements of a different animal, or by providing the buttock artwork BAW as a pair of pockets, the wearer may resemble an imaginary animal as worn. Preferably, the buttock artwork BAW does not include the torso, or other elements of the first animal extending beyond the bottom of the animal. By limiting the buttock artwork BAW as such, the integral appearance of the artwork worn on the wearer is enhanced.

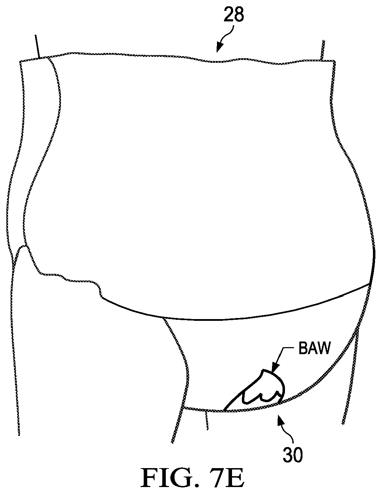

Referring to Figures 7D, the buttock artwork BAW is disposed in the back region 28, or disposed completely in the back region 28. Thus, the buttock artwork BAW has enhanced visibility when worn. When the buttock artwork BAW is disposed mainly in the crotch region 30, this may help distinguish the back and front side of the article prior to the article being worn on the wearer. However, when the buttock artwork BAW is disposed mainly in the crotch region 30, as in Figure 7E, the buttock artwork BAW may not enhance the integral appearance with the wearer as worn on the wearer. When the buttock artwork BAW is disposed mainly in the crotch region 30, it is hardly seen when worn on the wearer.

When the garment facing side of the back region 26 has a base color, the base color having an LAB value of $L_1$, $A_1$, and $B_1$, the portion of the buttock artwork BAW adjacent the base color having an outline color, the outline color having an LAB value of $L_2$, $A_2$, and $B_2$, the $\Delta E$ between the base color and the outline color is at least about 10, preferably from about 10 to about 50 based on the following formula, according to measurements herein.

$$\sqrt{(L_1 - L_2)^2 + (A_1 - A_2)^2 + (B_1 - B_2)^2}$$

The buttock artwork BAW may have a dimension of from about 30% to about 120%, or from about 40% to about 100%, of the transverse dimension of the absorbent material existing region 62. By providing the buttock artwork BAW in such transverse dimension, the integral appearance of the artwork with the wearer is enhanced.

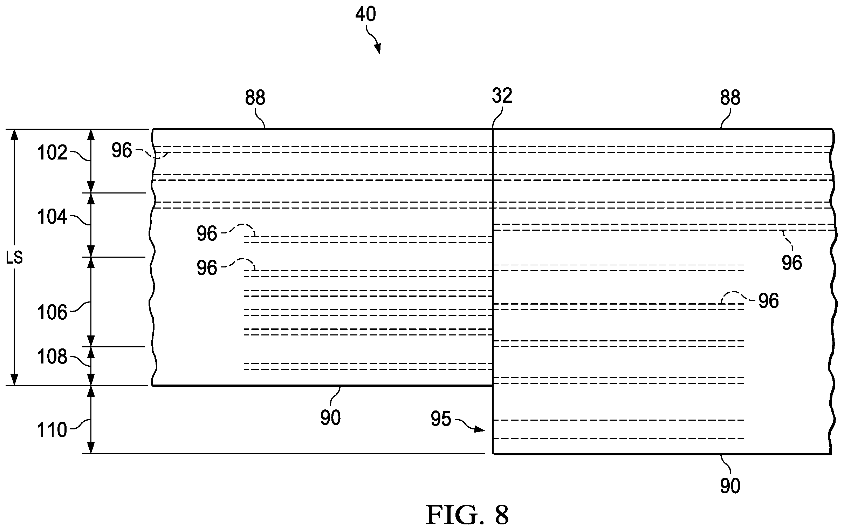

The article of the present invention may be that of the pant type as in Figure 5 wherein the application means is an elastic belt 40 extending transversely from the front and back regions 26, 28 of the absorbent chassis 38 and seamed with each other at the pair of transverse edges as side seams 32. Referring to Figure 8, elasticity of the elastic belt 40 may be provided by a plurality of elastic bodies 96 running in the transverse direction. The elasticity of the elastic bodies 96 may be inactivated in regions where the eye elements EE and buttock artwork BAW are superposed, such that the visibility or the artwork is not disturbed by the gathers created by the elasticity.

The artwork of the present invention may enhance the ergonomic fit perception of the pant type article. The elastic belt 40 has a proximal edge being located closer than a distal edge relative to the longitudinal center of the article. Referring to Figures 3A and 8, the front and back regions 26, 28 are each divided into 4 zones extending in the transverse direction and defined of its position from the distal edge 88 to the proximal edge 90 relative to the percentage of the seam length LS. In the example of Figure 8, the entirety of the length of the belt side edge of the front region 26 is the front belt 84, and is seamed with a certain length of the belt side edge of the back region 28 of the back belt 86 to define a seam length LS. When seam length LS is considered 0% at the distal edge 88 and 100% at the proximal edge 90 of the front belt 84, the zones are defined as such: 0-25% is the waist zone 102, 25-50% is the distal tummy zone 104, 50-85% is the proximal tummy zone 106, and 85-100% is the leg zone 108. When there is an elastic body disposed at 25% from the distal edge 88, such elastic body is considered to be included in the waist zone 102. When there is an elastic body disposed at 50% from the distal edge 88, or 85% from the distal edge 88, such elastic body is considered to be included in the proximal tummy zone 106. The eye elements EE are superposed with one of the front distal tummy zone 104 or the front proximal tummy zone 106. Referring to Figure 7C, by having the eye elements EE superposed with such zones, the integral appearance of the artwork with the wearer is enhanced. The buttock artwork BAW is superposed with one of the back distal tummy zone 104, back proximal tummy zone 106, or back leg zone 108, or in the back proximal tummy zone 106 or back leg zone 108. Referring to Figure 7D, by having the buttock artwork BAW superposed with such zones, the integral appearance of the artwork with the wearer is enhanced.

Referring to Figures 5, 7C, 7D, 8, and 9, the pant type article of the present invention takes a configuration of a belt type pant, wherein the application means is a ring-like elastic belt made of a front belt 84 and a back belt 86, wherein each of the front belt 84 and the back belt 86 have transversely continuous proximal and distal edges in parallel to the transverse axis. When the article takes a configuration wherein the back belt 86 has a greater longitudinal length LB than the longitudinal length LF of the front belt 84, the remaining length of "LB minus LS" of the back belt 86 is not counted in the 4 zones described above, but is defined as a back appendix zone 110. The buttock artwork BAW may be disposed completely in the back region 28 and the appendix zone 110.

In the article of the present invention, the tensile stress of the front proximal tummy zone 106 is made relative higher than the adjacent zones on the front, or higher than the tensile stress of any other zone, either in the front or the back. The absorbent material existing region 62 extends throughout the crotch region 30 and extend into the front region 26 up to the distal tummy zone 104, and further extend into the back region 28 up to the distal tummy zone 104 or the proximal tummy zone 106. The absorbent material existing region 62 may be positioned such that the front and back longitudinal edges of the absorbent material existing region 62 has the same distance from the distal edges of the front region 26 and the back region 28. Alternatively, the absorbent material existing region 62 may be positioned such that the front and back longitudinal edges of the absorbent material existing region 62 has a different distance from the distal edges in the front and back. The distance of the front distal edge to the absorbent material existing region 62 may be made shorter than that in the back.

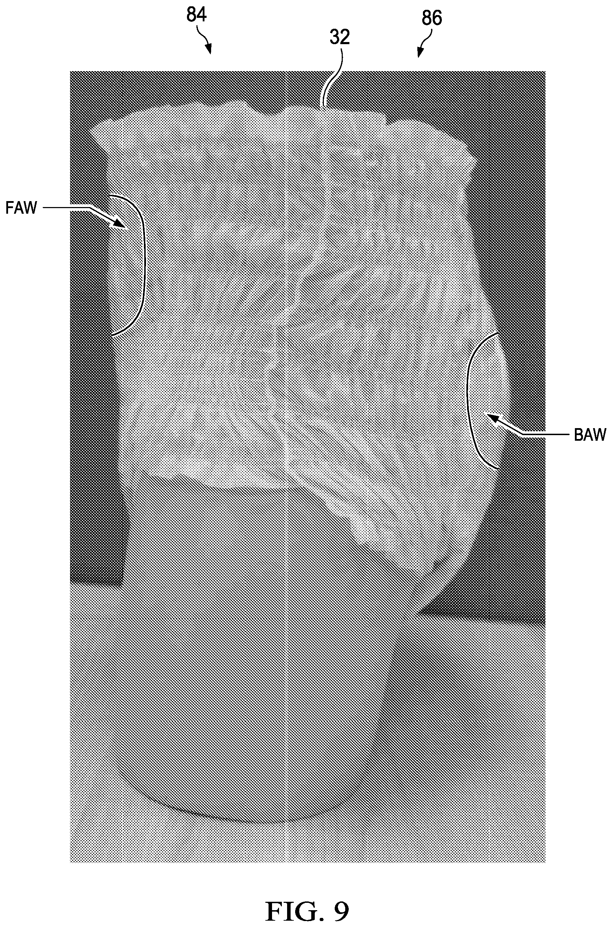

Without being bound by theory, such profiling of the tensile stress per zone and positioning of the absorbent material existing region 62 are believed to provide the article of the present invention with a shaped elastic belt 40 that conforms well to a human body, particularly to a young child of less than about 48 months of age, and therefore provide good fit and comfort to the wearer, without compromise of sagging prevention or leakage prevention. Namely, the front proximal tummy zone 106 is subject to high tensile stress such that the article may be anchored against the wearer's trochanter, while leaving more area for the back proximal tummy zone 106 to accommodate the wearer's buttock. As long as the article is anchored securely at the trochanter, the leg zone 108 adjacent the leg opening may be provided with significantly less tensile stress compared to the proximal tummy zone 106. Thus, the soft fit at the front leg opening region 120 facilitates leg movement. As a result of the profiling as described above, the article of the present invention may take an S-curve side seam 32 observed by the side when worn by the wearer, as shown as in Figure 9. Such S-curve side seam 32 may enhance the visibility of the side region 31 and enhance the three-dimension appearance of the artwork when worn. When the waist belt 40 of the present invention is measured against the Belt Seam Shape Measurement method described hereinbelow, the d value may be no less than +10mm, or no less than +15mm, or no less than +20mm. Such d value is indicative of the article conforming to the relatively greater front waist area and buttock area of the wearer, while providing good anchoring at the front proximal tummy zone 106. The curved side seam 32 and positive d value is observed no matter how the stretch board 180 is inserted in the sample, so long as a certain amount of time is allowed for the sample to reach equilibrium. For the article of Figure 9, the d value according to the Belt Seam Shape Measurement herein is positive. When the waist belt 40 of the article of the present invention is stretched to 70% of its full stretch, the seam 32 may be curved to the extent it has a width along the transverse axis of no less than +10mm, or no less than +15mm, or no less than +20mm.

The artwork of the present invention may be displayed as such wherein the eye elements EE are superposed with one of the front distal tummy zone or the front proximal tummy zone. The buttock artwork BAW may be superposed with one of the back distal tummy zone, back proximal tummy zone, or back leg zone, or superposed with one of the back proximal tummy zone, or back leg zone.

Dimension and Angle Measurements

For determining linear dimensions herein, the artwork or artwork elements are measured by a ruler, and according to its greatest dimension in a naturally contracted state before wear. For example, as in Figure 7A, the transverse dimension EEL of the eye elements EE is taken against its greatest transverse dimension.

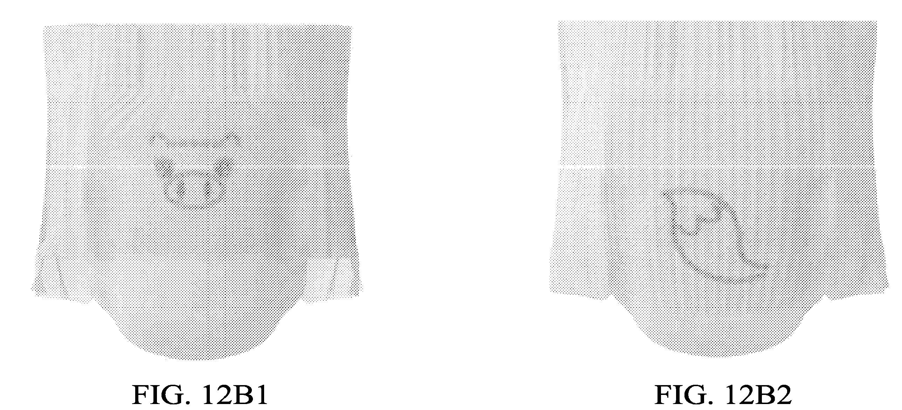

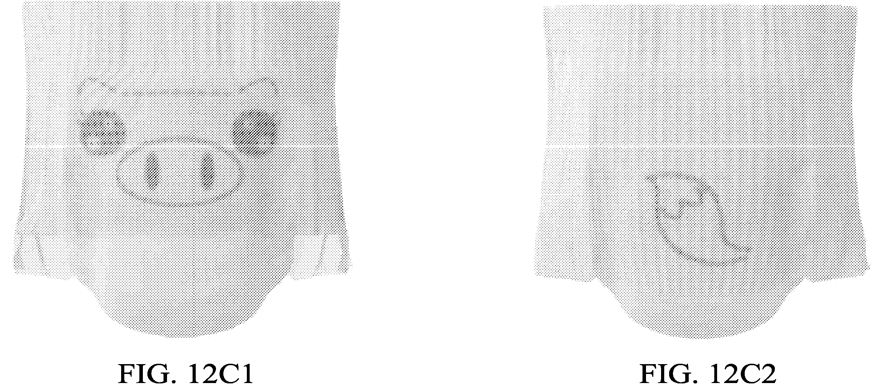

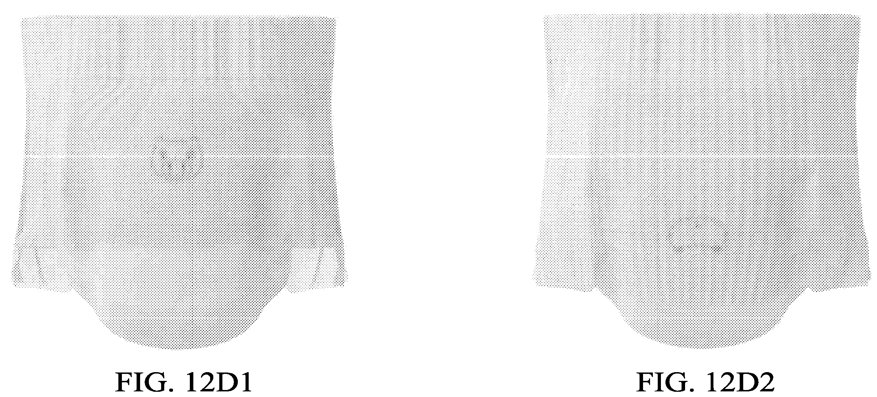

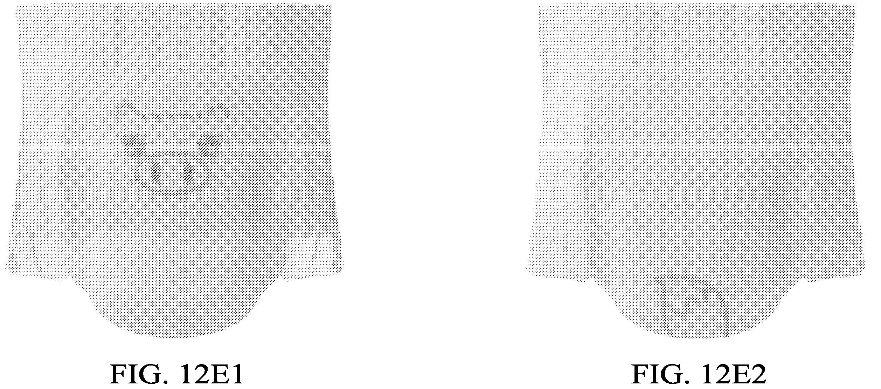

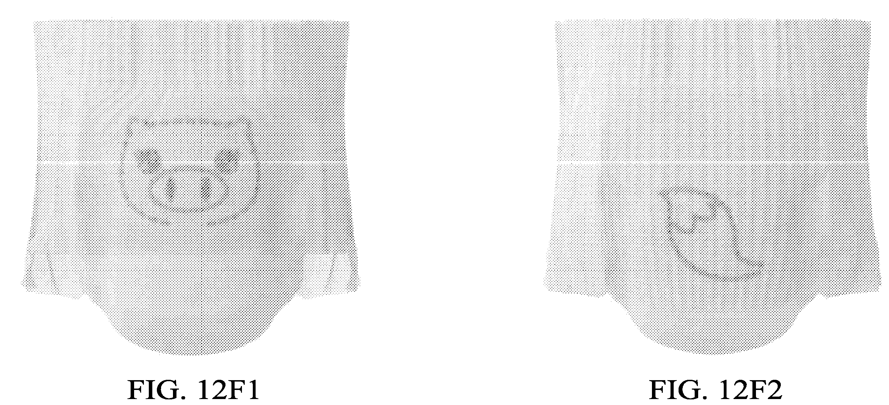

For determining the angle of the devoid area DA herein, the middle point of the eye elements EE are designated as the center point EEC. When the eye elements EE are disposed symmetrically in view of the longitudinal axis, the EEC is on the longitudinal axis, however, the EEC may not necessarily be on the longitudinal axis. The open areas of the contour artwork CAW are measured of its angle from EEC. The contour artwork CAW may be discontinuous, and thus there may be more than one devoid area DA. In such case, the devoid area DA of greatest angle is measured. (Multiple devoid area DAs are not accumulated.) For example, as in Figure 7A, the greatest devoid area DA towards the crotch region 30 is measured of its angle $\alpha$ relative to EEC. Small devoid areas which do not provide the impression of discontinuity, such as intervals between dotted lines or broken lines, are not considered devoid area DA. For example, in Figure 12F1, the opening toward the lower part of the front artwork FAW facing the crotch is a devoid area DA, however, the small openings between the broken lines are not considered devoid area DA.

Color Measurement

The article images are taken using a lab-built image acquisition system. The key components include a digital camera (Canon EOS 6D Mark 2 with an EF 24-105mm f/4L IS 2 USM lens or equivalent), a diffuse front light panel with a camera hole (DLP-600x600-WHI available from Smart Vision Lights, Muskegon, MI or equivalent) and a non-reflective black background plate. The articles are laid horizontally flat at the center of the black background plate. A calibrated color standard target containing 24 standard color chips (ColorChecker Passport, available from X'Rite Grand Rapids, MI or equivalent) are placed next to the article for color calibration. The camera is mounted right above the article at a distance of 105cm. The front light panel is under the camera with a distance of 90cm to the black background plate. The focal length of the camera is set to 105cm.

The camera white balance is calibrated by using the white color standard target of the X'Rite ColorChecker Passport and Canon EOS Utility software (version 3.9.0). "Custom white balance" menu is first selected and then click on the white target to calibrate the white balance. The image acquisition settings used are ISO: 100, F: 8.0, Exposure time: 1/100s, to achieve an image with proper exposure and contrast, such that there is no signal clipping in any of the color channels. The image is saved as .CR2 format (raw image for Canon camera) with a pixel dimension of 6240 by 4160 pixels. The resolution is 17 pixel/mm.

The image analysis of the artwork $\Delta E$ is conducted by the following steps:

a) The obtained raw images are converted to .dng file using the Adobe Light Room CC Version 8.1.

b) The generated .dng file is imported to the X'Rite ColorChecker Camera Calibration software version 1.1.1. The software automatically detects the ColorChecker Passport and generates a color profile (.dcp format) that can be imported into Adobe Light Room CC to calibrate the image color.

c) The generated .dcp color profile is applied to all the images in the Adobe Light Room CC to finish the color calibration process and saved as .tiff in RGB colorspace.

d) After the color calibration, the images are imported into the image j software (version 1.52h, National Institute of Health, USA) for converting the images from RGB color space to CIE Lab color space, assuming illuminant of D65.

e) A region of interest box of 10 × 20 pixels is used to select the representative area of two different features, for example, base color and eye color. The mean L, A, B values are obtained using the "histogram" module.

f) The $\Delta E$ is between the two features are calculated using the equation below:

$$\sqrt{(L_1 - L_2)^2 + (A_1 - A_2)^2 + (B_1 - B_2)^2}$$

where $L_1$, $A_1$, $B_1$ are the average L, A, B values of the first feature; $L_2$, $A_2$, $B_2$, are the average L, A, B values of the second feature.

Whole Article Force Measurement

This measurement is for pant type articles. Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at $23 \pm 2$ °C and $50 \pm 5$ % relative humidity.

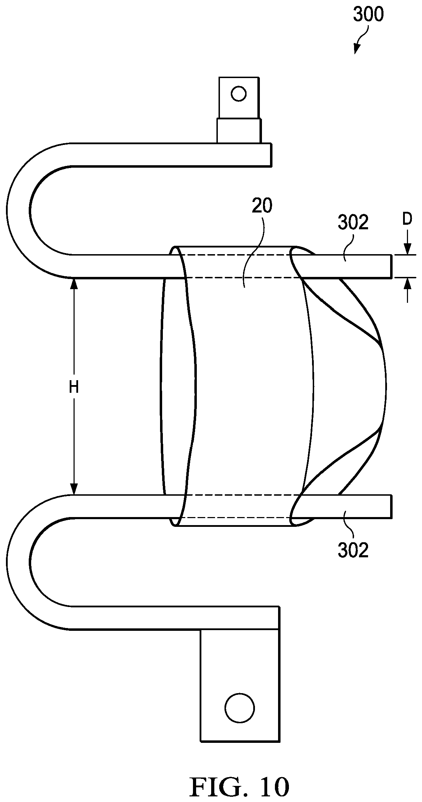

The tensile tester is fitted with hanger-type sample holding fixtures 300 as shown in Figure 10. Each fixture comprises a rigid linear rubber-coated horizontal bar section 302 to prevent sample slippage during testing. The outer bar diameter (including the rubber coating) of the horizontal bar sections is 10.0 mm. The central axes of the horizontal bar sections 302 are configured to remain parallel and in the same vertical plane throughout the test procedure. The gauge circumference is determined by the following equation:

$$\text{Gauge Circumference} = 2 \times (H + D + \pi D/2)$$

where H is the vertical gap between the horizontal bar sections 302, and D is the outer diameter of the bar.

The instrument is set up to go through the following steps:

| Crosshead Speed | 254.0mm/min |
| --- | --- |
| Final Load Point | 19.61 N |
| Hold Time | 0 |
| Number of Cycles | 1 |
| Data Acquisition Rate | 50Hz |

A sample article 20 is inserted onto the upper horizontal bar section 302 so that the bar passes through the waist opening and one leg opening of the article. The crosshead is raised until the specimen hangs above the lower bar and does not touch lower bar 302. The load cell is tared and the crosshead is lowered to enable the lower bar 302 to be inserted through the waist opening and other leg opening without stretching the article. The article is adjusted so that the longitudinal centerline L1 of the article is in a horizontal plane halfway between the upper and lower bars 302. The center of the side portion in contact with the bar 302 is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the initial gauge circumference at the same speed. The maximum circumference at 19.6N and the force at 70% stretch circumference during the extension segment of the test are recorded.

$$\text{Circumference (mm)} = 2 \times (H + D + \pi D/2)$$

The maximum circumference at 19.6N is defined as the Full Stretch Circumference (mm). The 70% stretch circumference is defined as the full stretch circumference $\times$ 0.7. The Waist Circumference Force is defined as the force at 70% stretch circumference during the load (extension) segment of the test.

Five samples are analyzed and their average Initial Gauge Circumference, average Full Stretch Circumference and average Waist Circumference Force are calculated and reported to the nearest 1 mm, 1 mm and 0.01 N, respectively.

Belt Zone Tensile Stress Measurement

The tensile stress (N/m) is calculated by tensile force (N) divided by the specimen width (m). Force may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10 and 90% of capacity of the load cell. The instrument is calibrated according to the manufacturer's instructions. All testing is performed in a room maintained at 23 ± 2 °C and 50 ± 5 % relative humidity. The instrument is equipped with single line contact grips at least as wide as the test specimen.

To obtain test specimens, the sample article is cut open along the side seams 32, and the front and rear elastic belt sections 40 are removed from the main body 38 by separating the bonding between the waist belt and main body. Cold Spray may be used, paying attention not to make wrinkles in the belt sections. Care is taken not to spray on any belt elastic body 96. The obtained elastic belts 40 are severed into zones 102, 104, 106, 108 according to the present invention with care not to cut any elastic body 96. Samples are pre-conditioned at 23 °C ± 2 C° and 50% ± 5% relative humidity for two hours prior to testing.

The instrument is set up to go through the following steps. Initial Gauge Length is calculated from the Initial Gauge Circumference which is determined during the Whole Article Force Test using separate identical articles, as described above. Initial Gauge Length = 0.5 × Initial Gauge Circumference. The final gauge length is calculated from the Full Stretch Circumference which is determined during the Whole Article Force Test, as described above.

| Crosshead Speed | 254.0 mm/min |
|---|---|
| Data Acquisition Rate | 50Hz |
| Final Gauge Length | 0.5 × Full Stretch Circumference |
| Hold Time | 0 |
| Number of Cycles | 1 |

One end of the specimen is clamped into the upper clamp and the load is tared. The other end of the specimen is clamped into the lower clamp. Approximately 5 mm of each end of the specimen is behind the contact line of the grip. The test is started and the specimen is extended to the final gauge length at a crosshead speed of 254 mm/min, then immediately returned to the original gauge length at the same speed. The specimen is extended in the article transverse direction during the test. The unload force at 70% of the Final Gauge Length during the unload segments of the test is recorded.

Five articles are analyzed and the unload forces are recorded for each of the front and back zones 102, 104, 106, 108. The average tensile force (N) is calculated to the nearest 0.01 N for each zone including the front and back specimens for that zone. The tensile stress for each zone is calculated by the average tensile force (N) divided by the average specimen width (m) and reported to the nearest 0.1 N/m.

Belt Seam Shape Measurement

A belt specimen from a pant type absorbent article 20 and a board for supporting the sample according to the size of the sample (hereinafter "stretch board") are prepared.

The belt specimen is prepared by removing the waist belt 40 from the main body 38 of the article by separating the bonding between the waist belt and main body. Cold Spray may be used, paying attention not to make wrinkles in the belt sections. Care is taken not to spray on any belt elastic body 96. The seam length LS (see Figure 8) of the sample is measured to within ± 1 mm with the belt laid flat and no tension applied.

The Full Stretch Circumference is determined during the Whole Article Force Test using different articles of the same batch, as described above. The Full Stretch Width is defined as 50% of the Full Stretch Circumference.

The stretch board 180 is made of polymethyl methacrylate, polycarbonate, or similar rigid material and has a dimension as such:

| Thickness | 8.5mm ± 5mm |
|---|---|
| Length | Between [the seam length (LS) + 40mm] to [the seam length (LS) + 100mm] |
| Width | 70% of the Full Stretch Width of the sample specimen - board thickness |

For example, if the belt side seam length LS is 130mm, the stretch board length should be 170-230mm. If the Full Stretch Width of the specimen is 355mm, and the board thickness is 8.5mm, the board width should be 240mm.

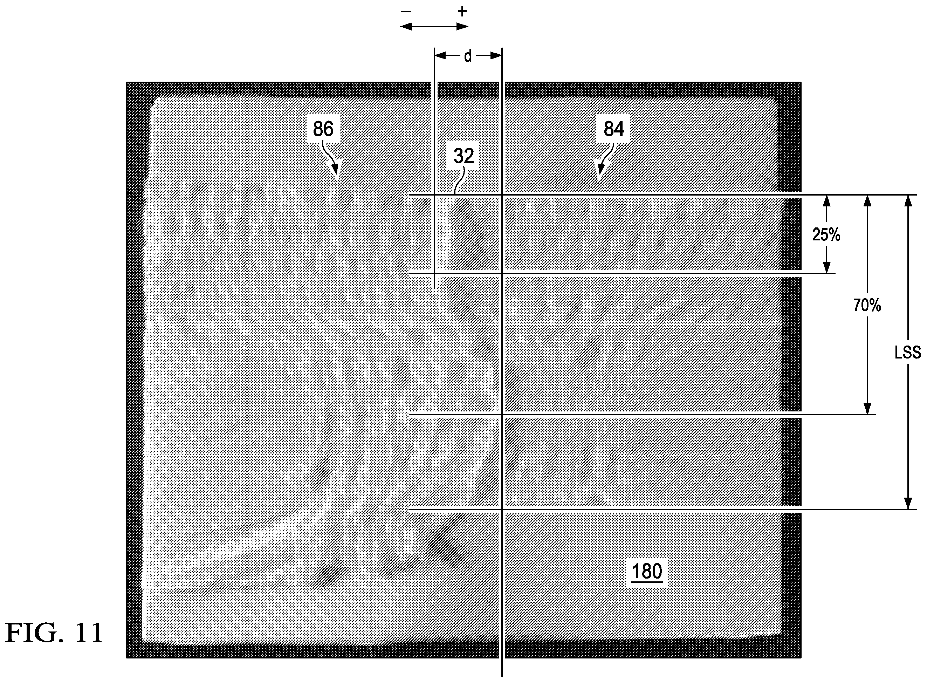

The stretch board 180 is inserted in the specimen while stretching the specimen as little as possible to insert the board, and in a manner such that the entire length of the seams 32 are placed on the front and back planes (and not on the sides) of the stretch board 180, such as shown in Figure 11. The specimen is adjusted on the stretch board 180 so that the distal edge 88 (i.e. at the waist opening) of each seam 32 on one side and the other of the stretch board 180, as well as the proximal edge (i.e. at the leg opening) of each seam 32 on one side and the other of the stretch board 180 are aligned to within $\pm$ 5 mm. respectively, of the same longitudinal axis.

The specimen with the stretch board 180 inserted is then stood for 1 min to reach equilibrium in an environment at 25 $\pm$ 2°C and 50 $\pm$ 10% RH. The linear end-to-end side seam length (rather than the contour length) in this stretched condition (LSS) is measured. The positions of the seam 32 in the transverse direction at points 70% of LSS away from the waist opening (70% point) and at 25% of LSS away from the waist opening (25% point) in the longitudinal direction are measured, and the difference "d" (unit: mm) is obtained (see Figure 11). The value d is positive when the 70% point is located closer to the front longitudinal centerline of the belt compared to the 25% point. The value d is negative when the 25% point is located closer to the front longitudinal centerline of the belt compared to the 70% point. The d value is obtained for both seams 32 on either side of the stretch board 180. The "d" values are measured for five identical articles and the average d value (average of 10 values) is reported to the nearest 1mm.

EXAMPLES

Examples 1 - 6 were created as a pair of synthetic photographs of pant type articles seen from the front and the back of the article, as in Table 1. What is meant by "EE Dimension" is the percentage of the eye elements relative to the transverse dimension of the absorbent material existing region. The synthetic photographs were created to present how the article appears in the worn state.

Table 1

|  | Figure Front/Back | EE Dimension | Other features |
|---|---|---|---|
| Example 1 | 12A1 / 2 | 60% | CAW has DA of 230 degrees |
| Example 2 | 12B1 / 2 | 45% | CAW has DA of 230 degrees |
| Example 3 | 12C1 / 2 | 110% | CAW has DA of 225 degrees |
| Example 4 | 12D1 / 2 | 20% | No DA |
| Example 5 | 12E1 / 2 | 60% | BAW in crotch region |
| Example 6 | 12F1 / 2 | 60% | CAW has DA of 30 degrees |

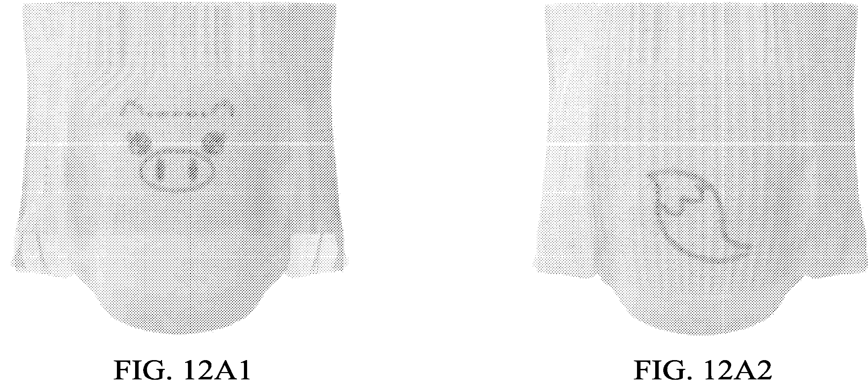

30 panelists who were caregivers of babies using pant diapers, mostly of Size 4 or 5 (L or XL size), and having a mixture of usage experience of major brands, were recruited. The panelists were shown front/back combination of visual presentations for Example 1 (Figures 12A1 and 12A2) and another front/back combination of visual presentations for one of Examples 2-6, and asked which one they preferred against the listed values. In Table 2, those questions yielding statistically significant preference (at confidence level of 90%) over the other is indicated by the Example number which was preferred. Those questions that yielded only statistically insignificant preference are indicated as "NS (not significant)".

Table 2

| Versus Example 1 | Example 2 | Example 3 | Example 4 | Example 5 | Example 6 |
|---|---|---|---|---|---|
| Overall preference | Example 1 | Example 1 | Example 1 | Example 1 | NS |
| 3D effect | Example 1 | NS | Example 1 | Example 1 | NS |
| Integral appearance | Example 1 | Example 1 | Example 1 | Example 1 | NS |
| Appealing Picture | Example 1 | NS | Example 1 | Example 1 | NS |
| Enable teaching moment | Example 1 | NS | Example 1 | Example 1 | Example 6 |

According to the test above, Example 1 which meets the requirements of the present invention have statistically significant overall preference over Examples 2-5 which do not meet the requirements of the present invention, or at the edge of the scope (Example 3). Example 6 which also meets the requirements of the present invention have similar preference compared to Example 1.

**Claims**

1. An absorbent article having a longitudinal direction and a transverse direction, the article having a front region (26), a back region (28), a side region (31), and a crotch region (30); the article comprising an absorbent chassis (38) and an application means;

   the absorbent chassis (38) comprising a liquid permeable topsheet, a liquid impermeable backsheet and an absorbent material existing region (62) disposed between the topsheet and the backsheet;
   the application means enabling the absorbent article to be applied by providing a waist opening and a pair of leg openings, the longitudinal dimension of the article matching the leg openings defined as the crotch region (30), wherein the remainder of the article is the front region (26) and the back region (28);
   wherein a facial artwork FAW visible from the garment facing side is disposed at least partially in the front region (26), the facial artwork FAW comprising a pair of eye elements EE disposed in the front region (26) superposed with the absorbent material existing region (62), wherein when worn by a wearer, the article provides an integral appearance of a three-dimensional article with the wearer by the front region (26) having the facial artwork FAW comprising eye elements EE, the side region (31), and the back region (28),
   wherein the application means is an elastic belt (40) extending transversely from the front and back regions of the absorbent chassis (38) and seamed with each other at the pair of transverse edges;
   wherein the eye elements EE are superposed with the absorbent material existing region (62) and have a dimension of from about 30% to about 120%, preferably from about 50% to about 110% of the transverse dimension of the absorbent material existing region (62), wherein the transverse dimension EEL of the eye elements EE is taken against its greatest transverse dimension;
   wherein the back region (28) comprises a buttock artwork BAW visible from the garment facing side disposed in the back region (28), wherein the buttock artwork BAW is superposed with the absorbent material existing region (62);
   wherein the elastic belt (40) has a proximal edge (90) being located closer than a distal edge (88) relative to the longitudinal center of the article, wherein the seam attaching the front region (26) and the back region (28) defines a seam length LS;
   the front and back regions each divided into 4 zones extending in the transverse direction and defined by its location from the distal edge (88) to the proximal edge (90) relative to the percentage of the seam length LS wherein; 0-25% is the waist zone (102), 25-50% is the distal tummy zone (104), 50-85% is the proximal tummy zone (106), and 85-100% is the leg zone; the optional remainder of the back region (28) is the appendix zone;
   wherein the absorbent material existing region (62) extends throughout the crotch region (30) and extends into the front region (26) up to the distal tummy zone (104), and further extends into the back region (28) up to the distal tummy zone (104) or the proximal tummy zone (106);
   wherein the tensile stress of the front proximal tummy zone (106) is relative higher than the adjacent zones on the front,
   wherein the eye elements EE are superposed with one of the front distal tummy zone (104) or the front proximal tummy zone (106), and the buttock artwork BAW is superposed with one of the back distal tummy zone (104), back proximal tummy zone (106), or back leg zone (108), preferably the buttock artwork BAW superposed with one of the back proximal tummy zone (106), or back leg zone (108); and
   wherein the d value according to the Belt Seam Shape Measurement herein is no less than about +10mm.

2. The article of Claim 1 wherein the buttock artwork BAW is a body element or a pair of pockets.

3. The article of Claim 1 or 2 wherein the facial artwork FAW is a first animal, and the buttock artwork BAW is a body element of a second animal, wherein the first animal and the second animal are different species.

4. The article of Claim 1 taking the configuration of a belt type pant, wherein the application means is a ring-like elastic belt (40) made of a front belt (84) and a back belt (86), wherein each of the front belt (84) and the back belt (86) have transversely continuous proximal and distal edges parallel to the transverse axis.

5. The article of any of Claims 1-4 wherein the facial artwork FAW and the buttock artwork BAW are printed on the backsheet.

**Patentansprüche**

1. Absorptionsartikel, der eine Längsrichtung und eine Querrichtung aufweist, wobei der Artikel einen vorderseitigen Bereich (26), einen rückseitigen Bereich (28), einen Seitenbereich (31) und einen Schrittbereich (30) aufweist; der Artikel umfassend eine Absorptionsgrundeinheit (38) und ein Anwendungsmittel;

   die Absorptionsgrundeinheit (38) umfassend eine flüssigkeitsdurchlässige Oberschicht, eine flüssigkeitsundurchlässige Unterschicht und einen Bereich (62), in dem Absorptionsmaterial vorhanden ist, der zwischen der Oberschicht und der Unterschicht angeordnet ist;

   wobei das Anwendungsmittel das Anwenden des saugfähigen Artikels durch ein Bereitstellen einer Taillenöffnung und eines Paars von Beinöffnungen ermöglicht, wobei die Längsabmessung des Artikels den Beinöffnungen entspricht, die als der Schrittbereich (30) definiert sind, wobei der Rest des Artikels der vorderseitige Bereich (26) und der rückseitige Bereich (28) ist;

   wobei ein Gesichtsbild FAW, das von der bekleidungsseitigen Seite sichtbar ist, wenigstens teilweise in dem vorderseitigen Bereich (26) angeordnet ist, das Gesichtsbild FAW umfassend ein Paar von Augenelementen EE, die in dem vorderseitigen Bereich (26) angeordnet sind, über dem Bereich (62), in dem Absorptionsmaterial vorhanden ist, wobei, wenn durch einen Träger getragen, der Artikel durch den vorderseitigen Bereich (26), der das Gesichtsbild FAW, umfassend Augenelemente EE, aufweist, den Seitenbereich (31) und den rückseitigen Bereich (28) ein einheitliches Erscheinungsbild eines dreidimensionalen Artikels für den Träger bereitstellt,

   wobei das Anwendungsmittel ein Gummibandbund (40) ist, der sich quer von dem vorderseitigen und dem rückseitigen Bereich der Absorptionsgrundeinheit (38) erstreckt und an dem Paar von Querrändern miteinander vernäht ist;

   wobei die Augenelemente EE über dem Bereich (62), in dem Absorptionsmaterial vorhanden ist, angeordnet ist, und eine Abmessung von etwa 30 % bis etwa 120 %, vorzugsweise von etwa 50 % bis etwa 110 % der Querabmessung des Bereichs (62), in dem Absorptionsmaterial vorhanden ist, aufweisen, wobei die Querabmessung EEL der Augenelemente EE im Verhältnis zu ihrer größten Querabmessung steht;

   wobei der rückseitige Bereich (28) ein Gesäßbild BAW umfasst, das von der bekleidungsseitigen Seite, die in dem rückseitigen Bereich (28) angeordnet ist, sichtbar ist, wobei das Gesäßbild BAW über dem Bereich (62), in dem Absorptionsmaterial vorhanden ist, angeordnet ist;

   wobei der Gummibandbund (40) einen proximalen Rand (90) aufweist, der sich relativ zu der Längsmitte des Artikels näher als ein distaler Rand (88) befindet, wobei die Naht, die den vorderseitigen Bereich (26) und den rückseitigen Bereich (28) verbindet, eine Nahtlänge LS definiert;

   wobei der vorderseitige und der rückseitige Bereich jeweils in 4 Zonen unterteilt sind, die sich in der Querrichtung erstrecken und durch ihre Position von dem distalen Rand (88) zu dem proximalen Rand (90) relativ zu dem Prozentsatz der Nahtlänge LS definiert sind, wobei; 0-25 % die Taillenzone (102) ist, 25-50 % die distale Bauchzone (104) ist, 50-85 % die proximale Bauchzone (106) ist und 85-100 % die Beinzone ist; der optionale Rest des rückseitigen Bereichs (28) die Anhangzone ist;

   wobei sich der Bereich (62), in dem Absorptionsmaterial vorhanden ist, durch den gesamten Schrittbereich (30) erstreckt und sich in den vorderseitigen Bereich (26) bis zu der distalen Bauchzone (104) erstreckt und sich weiter in den rückseitigen Bereich (28) bis zu der distalen Bauchzone (104) oder der proximalen Bauchzone (106) erstreckt;

   wobei die Zugspannung der vorderseitigen proximalen Bauchzone (106) relativ höher als die der angrenzenden Zonen auf der Vorderseite ist,

   wobei die Augenelemente EE über einer der vorderseitigen distalen Bauchzone (104) oder der vorderseitigen proximalen Bauchzone (106) angeordnet sind und das Gesäßbild BAW über einer der rückseitigen distalen Bauchzone (104), der rückseitigen proximalen Bauchzone (106) oder der rückseitigen Beinzone (108) angeordnet ist, vorzugsweise das Gesäßbild BAW über der rückseitigen proximalen Bauchzone (106) oder der rückseitigen Beinzone (108) angeordnet ist; und

   wobei der d-Wert gemäß der Messung der Bundnahtform hierin nicht weniger als etwa +10 mm beträgt.

2. Artikel nach Anspruch 1, wobei das Gesäßbild BAW ein Körperelement oder ein Paar von Taschen ist.

3. Artikel nach Anspruch 1 oder 2, wobei das Gesichtsbild FAW ein erstes Tier ist und das Gesäßbild BAW ein Körperelement eines zweiten Tieres ist, wobei das erste Tier und das zweite Tier unterschiedliche Arten sind.

4. Artikel nach Anspruch 1 in der Konfiguration einer Hose mit Bund, wobei das Anwendungsmittel ein ringförmiger Gummibandbund (40) ist, der aus einem vorderseitigen Bund (84) und einem rückseitigen Bund (86) besteht, wobei sowohl der vorderseitige Bund (84) als auch der rückseitige Bund (86) quere ununterbrochene proximale und distale Ränder parallel zu der Querachse aufweisen.

**5.** Artikel nach einem der Ansprüche 1 bis 4, wobei das Gesichtsbild FAW und das Gesäßbild BAW auf die Unterschicht gedruckt sind.

## Revendications

**1.** Article absorbant présentant une direction longitudinale et une direction transversale, l'article présentant une région avant (26), une région arrière (28), une région latérale (31), et une région d'entrejambe (30) ; l'article comprenant un châssis absorbant (38) et un moyen d'application ;

le châssis absorbant (38) comprenant une feuille supérieure perméable aux liquides, une feuille arrière imperméable aux liquides et une région existante de matériau absorbant (62) disposée entre la feuille supérieure et la feuille arrière ;

le moyen d'application permettant à l'article absorbant d'être appliqué en fournissant une ouverture de taille et une paire d'ouvertures pour de jambe, la dimension longitudinale de l'article correspondant aux ouvertures de jambe définies comme région d'entrejambe (30), dans lequel le reste de l'article est la région avant (26) et la région arrière (28) ;

dans lequel une illustration faciale FAW, visible depuis le côté faisant face au vêtement est disposée au moins partiellement dans la région avant (26), l'illustration faciale FAW, comprenant une paire d'éléments œillets EE, disposée dans la région avant (26) superposée à la région existante de matériau absorbant (62), dans lequel, lorsqu'il est porté par un utilisateur, l'article fournit une apparence intégrale d'un article tridimensionnel avec l'utilisateur par la région avant (26) présentant l'illustration faciale FAW, comprenant les éléments œillets EE, la région latérale (31), et la région arrière (28),

dans lequel le moyen d'application est une ceinture élastique (40) qui s'étend transversalement à partir des régions avant et arrière du châssis absorbant (38) et qui est cousue l'une à l'autre au niveau de la paire de bords transversaux ;

dans lequel les éléments œillets EE sont superposés à la région existante de matériau absorbant (62) et présentent une dimension d'environ 30 % à environ 120 %, de préférence d'environ 50 % à environ 110 % de la dimension transversale de la région existante de matériau absorbant (62), dans lequel la dimension transversale EEL des éléments œillets EE est prise par rapport à sa plus grande dimension transversale ;

dans lequel la région dorsale (28) comprend une illustration de fesse BAW visible depuis le côté faisant face au vêtement disposé dans la région arrière (28), dans lequel l'illustration de fesse BAW est superposée à la région existante de matériau absorbant (62) ;

dans lequel la ceinture élastique (40) présente un bord proximal (90) situé plus près qu'un bord distal (88) par rapport au centre longitudinal de l'article, dans lequel la couture fixant la partie avant (26) et la partie arrière (28) définit une longueur de couture LS ;

les régions avant et arrière chacune divisées en 4 zones s'étendant dans la direction transversale et définies par leur emplacement du bord distal (88) au bord proximal (90) par rapport au pourcentage de la longueur de couture LS dans lequel ; 0 à 25 % est la zone de taille (102), 25 à 50 % est la zone abdominale distale (104), 50 à 85 % est la zone abdominale proximale (106), et 85 à 100 % est la zone de jambe ; le reste facultatif de la région dorsale (28) est la zone d'appendice ;

dans lequel la région existante de matériau absorbant (62) s'étend à travers la région d'entrejambe (30) et s'étend dans la région avant (26) jusqu'à la zone abdominale distale (104), et s'étend en outre dans la région arrière (28) jusqu'à la zone abdominale distale (104) ou à la zone abdominale proximale (106) ;

dans lequel la contrainte de traction de la zone abdominale proximale avant (106) est relativement plus élevée que les zones adjacentes sur l'avant,

dans lequel les éléments oeillets EE sont superposés à une parmi la zone abdominale distale avant (104) ou la zone abdominale proximale avant (106), et l'illustration de fesse BAW est superposée à une parmi la zone abdominale distale arrière (104), la zone abdominale proximale arrière (106) ou la zone de jambe arrière (108), de préférence l'illustration de fesse BAW est superposée à une parmi la zone abdominale proximale arrière (106) ou la zone de jambe arrière (108) ; et

dans lequel la valeur d selon la mesure de forme de couture de la ceinture dans la présente description n'est pas inférieure à environ +10 mm.

**2.** Article selon la revendication 1 dans lequel l'illustration de fesse BAW est un élément de corps ou une paire de poches.

3. Article selon la revendication 1 ou 2, dans lequel l'illustration faciale FAW est un premier animal et l'illustration de fesse BAW est un élément corporel d'un second animal, dans lequel le premier animal et le second animal sont des espèces différentes.

4. Article selon la revendication 1 prenant la configuration d'un pantalon de type ceinture, dans lequel le moyen d'application est une ceinture élastique en forme d'anneau (40) constituée d'une ceinture avant (84) et d'une ceinture arrière (86), dans lequel la ceinture avant (84) et la ceinture arrière (86) présentent chacune des bords proximaux et distaux transversalement continus parallèles à l'axe transversal.

5. Article selon l'une quelconque des revendications 1 à 4, dans lequel l'illustration faciale FAW et l'illustration de fesse BAW sont imprimés sur la feuille arrière.

FAW

CAW    EE  NE    CAW
          ME    EE

192

**FIG. 1A**

26

31    190AW  190AW

30

**FIG. 1B**

FIG. 2A

FIG. 2B

EP 3 906 001 B1

FIG. 3A

FIG. 3B

EP 3 906 001 B1

FIG. 4B

FIG. 4A

20

82    38    88

32

84

40

86

28

26    32

42    90

30    48

38

FIG. 5

26AW

26AW

EE  ME  NE  EE

FAW

30AW

## FIG. 6A

BAW

## FIG. 6B

FIG. 7A

FIG. 7B

FIG. 7C

FIG. 7D

28

BAW

30

FIG. 7E

FIG. 8

FIG. 9

FIG. 10

FIG. 11

FIG. 12A2

FIG. 12A1

FIG. 12B2

FIG. 12B1

FIG. 12C2

FIG. 12C1

FIG. 12D2

FIG. 12D1

FIG. 12E2

FIG. 12E1

FIG. 12F2

FIG. 12F1

**EP 3 906 001 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0035401 A1
- EP 2596715 A1
- EP 1139955 A1
- WO 2018152831 A1